# EUROPEAN PATENT APPLICATION

(11) **EP 4 091 615 A1**
(43) Date of publication of application: **23.11.2022**
(21) Application number: 21174941.1
(22) Date of filing: 20.05.2021
(51) Int. Cl.: A61K 31/56, A61P 1/04, A61K 9/46, A61K 9/00, A61K 9/20

(54) **ORODISPERSIBLE EFFERVESCENT TABLET COMPRISING BUDESONIDE FOR USE IN THE TREATMENT OF EOSINOPHILIC ESOPHAGITIS**

(71) Applicant: Dr. Falk Pharma GmbH, 79108 Freiburg (DE)
(72) Inventor: WILHELM, Rudolf, 76476 Bischweier (DE); MÜLLER, Ralph, 79111 Freiburg (DE); GREINWALD, Roland, 79431 Kenzingen (DE); PRÖLS, Markus, 79100 Freiburg/Breisgau (DE)
(74) Representative: Lederer & Keller Patentanwälte Partnerschaft mbB

(57) **Abstract**

Orodispersible effervescent tablets comprising budesonide are administered to patients suffering from eosinophilic esophagitis whereby a complete mucosal healing is achieved by a dosage regimen of administering said orodispersible effervescent tablets for at least six weeks to twelve weeks for the induction period and thereafter up to three years as maintenance treatment.

## Description

Eosinophilic esophagitis (EoE) is conceptually defined in the revised American Gastroenterological Association's consensus recommendations for diagnosis and treatment of EoE as a "chronic, antigen-/immune-mediated esophageal disease characterized by an eosinophil-predominant inflammation of the esophageal mucosa causing symptoms of esophageal dysfunction". Currently, EoE is defined by the combination of clinical symptoms of esophageal dysfunction, most commonly dysphagia and chest pain, and the histological finding of at least 15 eosinophils (eos) in one high power microscopic field (hpf) from a biopsy from the esophageal mucosa and should be diagnosed by clinicians, taking into consideration all clinical and pathologic information; neither of these parameters should be interpreted in isolation.

Eosinophilic esophagitis is mainly found in industrialized countries such as Europe, the United States, Canada, and Australia, with an increasing prevalence and incidence. In a recent systemic review by Navarro P, Arias A, et al. Aliment Pharmacol Ther 2019;49:1116-25, the pooled prevalence of EoE was 34.4 cases per 100 000 inhabitants (95% CI, 23.1-47.5), with pooled EoE incidence rates of 6.6/100 000 person-years (95% CI, 3-11.7) in children and 7.7/100 000 (95% CI, 1.8-17.8) in adults. Males are more commonly affected than females, accounting for about 76% of adult patients and 66% of pediatric patients. Eosinophilic esophagitis affects all age groups with a peak between the age of 20 and 50.

In adults, the most common presenting clinical symptoms are intermittent dysphagia for solids (range: 29%-100% depending on the series) and food impaction (range: 25%-100%). One report found that EoE was responsible for 50% of cases of esophageal food impaction in one institution. Gastro-Esophageal-Reflux-Disease (GERD)-like symptoms are also reported (range: 7%-100%). Chest pain that is unrelated to swallowing activity (range: 1%-58%), abdominal pain (range: 3%-25%); diarrhea and weight loss are reported in some patients. Many adults had long-standing symptoms including recurrent food impactions prior to the diagnosis of EoE.

Eosinophilic esophagitis tends to be a chronic disease with persistent or relapsing symptoms, but is not characterized by mucosal ulceration or destruction and does not appear to limit life expectancy. To date, esophageal strictures and small caliber esophagus, often resulting in trouble of swallowing (i.e., dysphagia) up to food impaction, have been the major complications identified. When these findings are encountered, either radiologically or at endoscopy, a high index of suspicion should be raised for EoE, and mucosal biopsy specimens should be obtained. Careful long-term follow-up is advised. Other chronic problems include failure to thrive and feeding intolerance in children. However, if left for longer time untreated, strictures develop by esophageal remodeling.

Dellon et al., Gastroenterology, 2012, 143, pp 321-324 performed a randomized trial to compare nebulized and then swallowed (NEB) versus viscous topical corticosteroid (OVB) treatments for eosinophilic esophagitis (EoE). The patients received budesonide 1 mg twice daily either nebulized and then swallowed (NEB) or as an oral viscous slurry (OVB) for eight weeks. After treatment, eosinophilic counts markedly improved in the oral viscous slurry treated group but not in the nebulized and then swallowed group. The study illustrates the importance of adequate esophageal targeting for efficacy.

A highly favourable effect of systemic and topical corticosteroids has been demonstrated for systemic and topical corticosteroids. However, treatment with corticosteroids is associated with side effects. Long-term treatment with systemic corticosteroids is burdened with severe adverse effects, mainly depending on the duration and the dosage of the medication.

In contrast, budesonide is acting dominantly locally due to its affinity to the tissue and its high grade first pass liver metabolism, and showed a positive benefit/risk ratio in EoE. However, it is unclear, whether long-term use is able to maintain clinico-pathological remission.

As already shown by Dellon et al., Gastroenterology, 2012, the mucosal medication contact time, measured by scintigraphy, was higher for an oral viscous budesonide suspension group (OVB) than the nebulized and then swallowed budesonide (NEB) group (P < .005). OVB provided a significantly higher level of esophageal exposure to the therapeutic agent, which correlated with lower eosinophil counts. With this oral viscous slurry formulation and a budesonide 1mg twice daily application, 64% of patients versus 27% under NEB achieved complete histological remission (i.e., <1 eosinophil/high power field), mainly due to prolonged contact time.

Greuter et al. (American Journal of Gastroenterology, October 2017, Vol. 112, Issue 10, pp 1527-1535 describes long-term treatment of eosinophilic esophagitis with swallowed topical corticosteroids. In this study corticosteroids prepared for the treatment of asthma were used off-label as viscous corticosteroid solutions. Viscous suspensions containing 1.0 mg fluticasone or budesonide were administered twice daily. It has been reported that a deep remission was achieved after 89 weeks of treatment.

It turned out that orodispersible tablets such as described in EP 2 886 108 and EP 3 086 782 are pharmaceutically well-accepted formulations suitable for esophageal targeting which provides small amounts of budesonide during the dissolution in the mouth during a period of about 2 minutes up to 20 minutes, preferably about 2-5 minutes to the esophagus. The embodiments described in the two European patents are preferably used according to the present invention.

In the present invention the budesonide is preferably administered as an orodispersible effervescent tablet which contains 0.5 mg or 1.0 mg or 2.0 mg budesonide. The effervescent action is caused by the salt of at least one pharmaceutically acceptable acid which in an aqueous environment, in particular in the mouth containing saliva, can release a gas which is preferably CO₂ in cooperation with a further acid. The effervescent tablet contains the salt of a further weak acid or a further weak acid that decreases the pH value in the aqueous solution. The preferably used orodispersible effervescent tablet has a mass between 100 mg to 200 mg, and preferably between 130 mg to 150 mg. In a preferred embodiment the orodispersible effervescent tablet contains sucralose instead of aspartam in an amount of about 0.1 to 1.0% by weight based on the weight of the orodispersible effervescent tablet. The salt of the pharmaceutically acceptable acid can release a gas in the aqueous (saliva) environment of the mouth together with an acid. An essential component of the orodispersible effervescent tablet is at least one of the carbonates selected from the group consisting of NaHCO₃, Na₂CO₃, KHCO₃, K₂CO₂ and CaCO₃ or mixtures thereof. The salt of the further pharmaceutically acceptable weak acid that decreases the pH value in the aqueous (saliva) solution is disodium citrate, monosodium citrate or a mixture thereof. The orodispersible effervescent tablet which is preferably used according to the teaching of the present invention is laid on the tip of the tongue and it dissolves slowly driven by the reaction between the saliva and the effervescent system. The tablet is comparatively small and has a diameter of about 5-10 mm and a height of 1.5-3.0 mm. The tablets are prepared by applying a suitable pressure in the tableting process such that the fracture strength is between about 10 N to 100 N and the friability is at most 5%.

According to the present state of medical research it turned out that eosinophilic esophagitis (EoE) is a chronic, inflammatory disease of the esophagus. For diagnosis of EoE the following criteria are considered:
- presence of symptoms of esophageal dysfunction and predominantly eosinophilic inflammation of the esophagus;
- endoscopic evaluations, in particular with regard to a number of mucosal alterations regarding EoE associated endoscopic signs like edema, rings, exudates, longitudinal furrows and/or strictures.

The eosinophil is a late-phase inflammatory cell that plays an important role in tissue repair and remodeling. It turned out that eosinophilic inflammation may induce fibrosis with the consequence of luminal narrowing and stricture formation finally resulting in an alteration of esophageal functions. It is assumed that EoE is a progressive fibrostenotic disease since there are indications that the extent of fibrosis has been positively associated with disease duration. As the disease progresses over time, a mixture of inflammatory and fibrotic features can be observed.

One potential diagnostic means for assessing disease activity and disease progression that provides information on esophageal remodeling is endoscopic imaging. Characteristic esophageal abnormalities which can be evidently seen on endoscopy in EoE include:
- edema (loss of vascular markings)
- rings (trachealization)
- exudates (white plaques)
- longitudinal furrows (vertical lines) and
- strictures.

These features can be assessed and graded in their presence and intensity by using the validated endoscopic reference score (called EREFS) as being published by Hirano et al. Gut. 2013;62(4):489-95.

### Major features:

### Fixed rings (also referred to as concentric rings, corrugated esophagus, corrugated rings, ringed esophagus, trachealisation)

- Grade 0: none
- Grade 1: mild (subtle circumferential ridges)
- Grade 2: moderate (distinct rings that do not impair passage of a standard diagnostic adult endoscope (outer diameter 8 - 9.5 mm)
- Grade 3: severe (distinct rings that do not permit passage of a diagnostic endoscope)

### Exudates (also referred to as white spots, plaques)

- Grade 0: none
- Grade 1: mild (lesions involving ≤10% of the esophageal surface area)
- Grade 2: severe (lesions involving >10% of the esophageal surface area

### Furrows (also referred to as vertical lines, longitudinal furrows)

- Grade 0: absent
- Grade 1: present

### Edema (also referred to as decreased vascular markings, mucosal pallor)

- Grade 0: absent (distinct vascularity present)
- Grade 1: loss of clarity or absence of vascular markings

### Stricture

- Grade 0: absent
- Grade 1: present

### Minor feature:

Crêpe paper esophagus (mucosal fragility or laceration upon passage of diagnostic endoscope but not after esophageal dilation).
- Grade 0: absent
- Grade 1: present
The total score ranges from 0-9 points.

A total EREFS score of '0 points' defines 'deep endoscopic remission', i.e. the complete absence of any inflammatory or fibrostenotic signs of EoE. The endoscopic assessment of the esophageal features of eosinophilic esophagitis is measured according to the teaching of Hirano et al. A deep endoscopic remission according to the present invention is obtained when a total EREFS score of 0 points is achieved. Therefore, a high standard is applied to the deep endoscopic remission whereby all the features as lined out above must meet a score of 0 points.

Since the endoscopic determination allows only a visual inspection of mucosal inflammatory and fibrotic signs, but is not suitable to detect changes in organ function of the esophagus itself, an objective and measurable system can be used complementary to endoscopy to study changes in esophagus function. The Endolumenal Functional Lumen Imaging Probe (EndoFLIP^{®}, Crospon Ltd., Galway, Ireland) is a commercial lumen imaging catheter, which is emerging as a new standard for assessment of luminal dimensions and compliance. The balloon catheter is deployed alongside an endoscope during patient evaluation, and inflated with fluid to a low distending pressure of 40 mmHg or up to 60 mL. The device contains a cylindrical bag that can be inflated with a conductive fluid while simultaneously CSA (cross-sectional area) and intra-bag pressure and applied volume are measured to calculate the distensibility of the esophagus which mimics its function.

It is assumed that the distensibility evaluation of the esophagus using the functional lumen imaging probe (FLIP) offers an objective measure to characterize fibrostenotic remodeling processes in patients with EoE and the effect on these fibrostenotic remodeling processes under treatment with the present invention. Left untreated, the chronic inflammation in EoE is believed to progress to fibrostenotic changes manifested as the characteristic ringed and strictured esophagus that can result in esophageal mechanical obstruction and associated symptoms, in particular dysphagia.

The therapeutic success of achieving deep endoscopic remission can be measured by the modified EREFS score of '0 points' and its physiological consequence as an increased esophageal distensibility as measured by the EndoFLIP^{®}.

The present invention provides a dosage regimen for an orodispersible effervescent tablet comprising budesonide as described herein for use in the treatment of eosinophilic esophagitis wherein a deep endoscopic remission and deep histological remission is achieved by the twice daily administration of the orodispersible effervescent tablet for a duration of at least six weeks (42 days) and also maintained over a period of at least 48 weeks. This is designated in the sense of the invention as complete mucosal healing. According to a preferred embodiment of the present invention the dosage regimen comprises an induction period and a maintenance period. Surprisingly, during the induction period of a duration of 6-12 weeks complete mucosal healing is already achieved. During the induction period the patient is treated with orodispersible effervescent tablets as described herein whereby twice daily, preferably in the morning and in the evening a tablet comprising 1 mg budesonide is administered. In an alternative embodiment the induction treatment can be obtained with an once daily administration of an orodispersible effervescent tablet containing 2 mg budesonide.

After the induction period a maintenance period follows which lasts from 48 weeks up to 3 years. During the maintenance period the orodispersible effervescent tablet is preferably administered twice daily with 1 mg budesonide contained within each tablet. In another embodiment an orodispersible tablet containing 0.5 mg budesonide is administered twice daily. In an alternative embodiment the maintenance treatment can be obtained with an once daily administration of an orodispersible effervescent tablet containing either 1 mg or 2 mg budesonide.

One of the most remarkable effects obtainable with the present treatment regimen is that fibrostenotic changes in the esophagus can be avoided and in part reverted. This is an important aspect since as soon as the esophagus has entered in a conversion of the tissues, treatment of the patient may become very difficult and longlasting symptoms of esophageal dysfunction including frequent bolus impaction might occur.

Another surprising effect is that complete mucosal healing can be obtained after a short induction period and be maintained over a long time span.

The dosage regimen of the present invention prescribes that an orodispersible effervescent tablet is administered to the patient in doses of 0.5 mg or 1.0 mg budesonide twice a day or 2 mg budesonide once daily. The application is preferably applied in a format wherein 1 mg budesonide is applied in the form of one tablet comprising 1 mg budesonide twice daily. Alternatively, two tablets comprising 0.5 mg budesonide in the morning and one tablet comprising 1 mg budesonide or two tablets comprising 0.5 mg budesonide in the evening can be administered. The initiation treatment has to be performed over a period of at least six weeks (42 days) up to 12 weeks (84 days) for induction of deep endoscopic remission including complete mucosal healing. It follows a maintenance treatment as decided by the treating physician for a duration of 48 weeks up to 3 years.

The disintegration of each orodispersible effervescent tablet in the mouth occurs in a time span of at least 2 minutes to about 10 minutes, preferably in a time span extending from at least 2 minutes to about 5 minutes, but can take up to 20 minutes. This delayed disintegration of the orodispersible effervescent tablet results in a continuous production of saliva in which budesonide is suspended during disintegration of the present invention. During this prolonged disintegration time, the patient swallows on average 10-times the budesonide containing saliva. It is assumed that the naturally occurring mucoadhesive agents in the saliva result in a stable coating of the esophagus whereby the budesonide is brought into close contact to the cells of the outer surface of the esophagus whereby an uptake of budesonide can take place.

Up to now it was assumed that stopping and reverting the fibrostenotic remodelling is a lengthy process, which needs time. Therefore, it was unclear whether a drug can quickly work on this and is able to induce and in addition to maintain this deep endoscopic remission over a longer period. Achieving such a deep endoscopic remission state should increase the likelihood of stopping the fibrostenotic remodelling and even to revert this, thus an improvement of esophageal distensibility towards normal values might be possible.

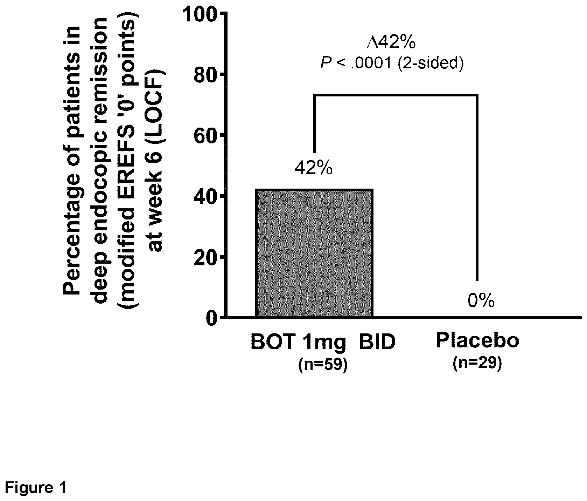

Surprisingly, the dosage regimen, namely an at least 6-week treatment with budesonide 1 mg orodispersible effervescent tablet twice daily in clinico-histological active EoE patients was also able to bring significantly more patients into deep endoscopic remission compared to placebo, as shown in Figure 1.

Figure 1 is a summary of the results observed in the EOS-1 clinical trial described further in the examples. At baseline of the induction of remission treatment, none of the patients was in deep endoscopic remission. It turned out that a treatment with orodispersible effervescent tablets comprising 1 mg budesonide administered twice a day (BID) had the consequence that 42% of the patients achieved deep endoscopic remission at week 6 (42 days after start of the treatment) compared with 0% of patients who received a placebo.

This unexpected rapid success of targeted treatment with achievement of deep endoscopic remission mirrors also the observed quick and high rates (89.8%) of deep histological remission (i.e., '0' eos/hpf in all biopsies) observed under a 6-week treatment with budesonide 1 mg orodispersible effervescent tablet twice daily compared to 0% under placebo.

This rapid onset and the extraordinary efficacy might be well explained by the special pharmaceutical formulation, its release and esophageal targeting profile of the present invention.

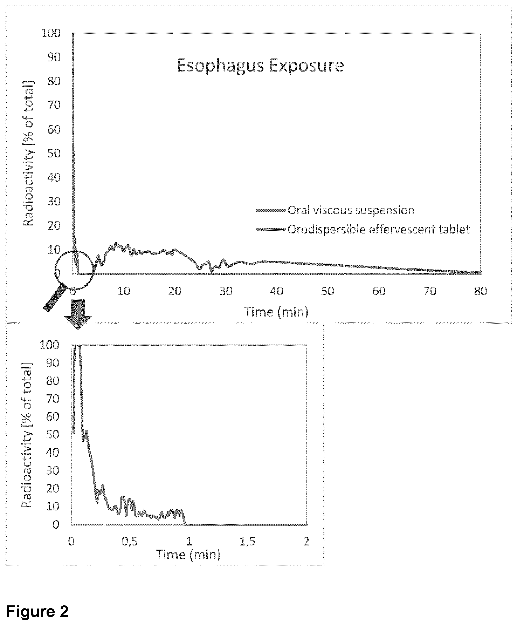

In a recent scintigraphic study it could surprisingly be shown that an ¹¹¹Indium-labelled orodispersible effervescent tablet comprising 1 mg budesonide provides a substantially longer retention of the radioactivity (and thus also of the budesonide) in the esophageal mucosa than 5ml of an ¹¹¹Indium-labelled oral viscous budesonide suspension [0.2mg/ml]. A representative picture of the percentage of radioactivity observed over time in the esophagus is shown in Figure 2. The study was performed with healthy volunteers (N=10). Each volunteer obtained each of the two preparations whereby the distribution of the indium was observed with a suitable scintigraphic camera. As particular regions of interest the oral/buccal area, the esophagus and the stomach were defined. At different periods of time the radioactivity in the esophagus was set in relation to the total radioactivity.

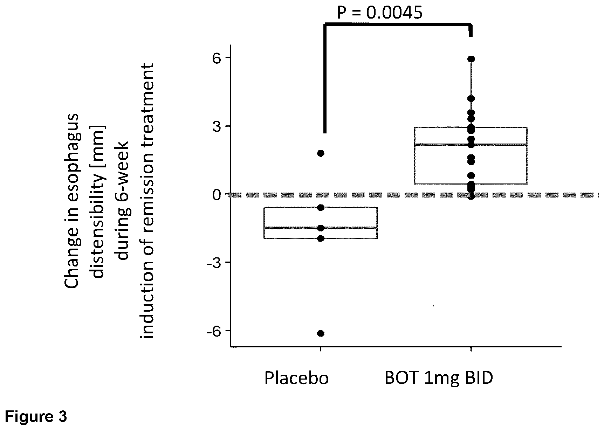

In addition, and also not expected due to the short treatment period of only 6 weeks, the esophageal distensibility (i.e., the diameter of the esophagus) also significantly improved compared to placebo as depicted in Figure 3.

Figure 3 shows the change from baseline in the distensibility of the esophagus [measured in mm]. The values were measured 42 days after start of the treatment and measured by the EndoFLIP test system. It turned out that under placebo treatment the distensibility of the esophagus decreased (indicating a narrowing of the esophageal diameter), whereas the distensibility in the group obtaining the invention (orodispersible effervescent tablet comprising 1 mg budesonide administered twice daily) was significantly improved (indicating a widening of the esophageal diameter).

This significant improvement in the esophagus diameter within such a short time of only 6-weeks could only be explained by the extremely high local anti-inflammatory activity of the budesonide orodispersible effervescent tablet. This is caused by the mode of release and delivery of budesonide from the orodispersible effervescent tablet to the esophageal mucosa and its prolonged contact time to the mucosa.

In addition, and unexpected due to the short treatment period of only 6 weeks, the new dosage regimen of budesonide 1mg orodispersible effervescent tablet formulation when administered twice daily led to complete histological remission rates of 90%. Again, this is caused by the mode of release and delivery of budesonide from the orodispersible effervescent tablet to the esophageal mucosa and its prolonged contact time to the mucosa.

Beside the unexpected high efficacy in improvement of the esophageal distensibility as well as in the induction of deep endoscopic remission, both achieved already after only a 6-week treatment course with budesonide 1 mg orodispersible effervescent tablets (twice daily). Recent long-term data showed that budesonide 1 mg or 0.5 mg orodispersible effervescent tablets (twice daily) were able to maintain such effects also over a period of 48 weeks in a placebo-controlled double-blind trial, as presented in Table 1 below:

**Table 1: Course of "deep endoscopic remission" over 48-weeks maintenance treatment (FAS-DB)**

| | **Proportion of patients with deep endoscopic remission during a 48-weeks maintenance treatment** | | |
|---|---|---|---|
| | BOT 0.5mg BID | BOT 1.0mg BID | Placebo |
| | n=68 | n=68 | n=68 |
| **Week 0** | 34 (50.0%) | 34 (50.0%) | 35 (51.5%) |
| **Week 48** | 36 (52.9%) | 39 (57.4%) | 4 (5.9%) |

| | | | |
|---|---|---|---|
| *BID =̂ twice daily | | | |

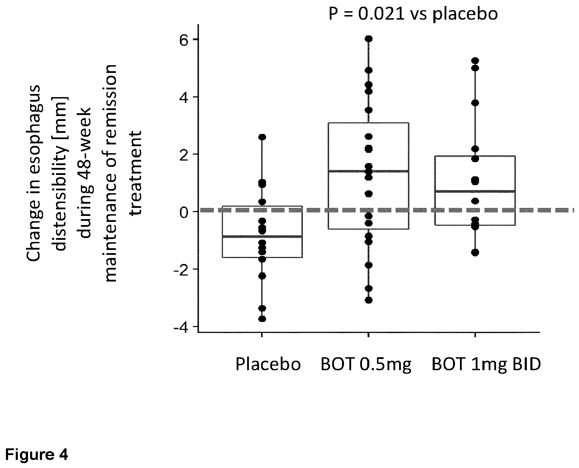

In addition, the esophageal distensibility (i.e., the diameter of the esophagus) also significantly further improved under treatment with budesonide orodispersible effervescent tablets over 1-year maintenance treatment compared to placebo (p=0.021) as shown in Figure 4. Although the distensibility of the esophagus further improved also with the lower dosage of 0.5 mg budesonide it should be mentioned that the statistic variance of the values obtained with 0.5 mg budesonide is substantially higher at a treatment of 0.5 mg budesonide twice daily. Therefore, it is preferred to administer orodispersible effervescent tablets (BUL) containing 1 mg budesonide twice daily (BID).

Surprisingly, during a prolonged 2-years open-label extension maintenance treatment with budesonide orodispersible effervescent tablets, only 2 of 166 patients (1.2%) experienced a clinical relapse, or a food impaction which needed endoscopic intervention or need an endoscopic dilation. This unexpected change in the natural course of the disease was also reflected by the fact that the rate of achieved deep endoscopic remission at the beginning of this 2-year open-label treatment phase (55 out of 82 patients with an endoscopy (67.1%)) did not changed during this 2-year treatment phase. Even more, it was a surprise that by a total treatment of 3 years with budesonide orodispersible effervescent tablets it was possible to maintain deep histological remission, i.e. ,0' eos/mm² hpf in about 40 of 49 patients (81.6%) and to maintain deep endoscopic remission in about 38 of 50 patients (76.0%).

### Examples

The results as shown in Figures 1-3 were obtained by clinical trials which were performed as follows:

### Example 1

### EOS-1 induction of remission

**BACKGROUND & AIMS:** This trial was a randomized, placebo-controlled trial to assess the effectiveness and tolerability of an orodispersible effervescent tablet comprising budesonide (BOT; now called ,budesonide orodispersible tablet') formulation, i.e., the present invention, which allows the drug to be delivered to the esophagus in adults with active EoE.

**METHODS:** EOS-1 was a pivotal, randomized, double-blind induction of remission trial in 88 adult EoE patients performed in several European countries. Patients were randomly assigned to groups that received BOT (1 mg twice daily; n=59) or placebo (n=29) for 6 weeks. The primary end point was complete remission, based on clinical and histologic factors, including dysphagia and odynophagia severity ≤2 points on a scale of 0-10 (,0' = no symptoms; ,10' = most severe symptoms) on each of the 7 days before the end of the double-blind phase and a peak eosinophil count <5 eosinophils/high power field. Patients who did not achieve complete remission at the end of the 6-week double-blind phase were offered 6 weeks of open-label treatment with BOT (1 mg twice daily).

**RESULTS:** At 6 weeks, 58% of patients given BOT were in complete clinical and histological remission compared with no patients given placebo (P < .0001). The secondary end point of histologic remission was achieved by 93% of patients given BOT vs no patients given placebo (P < .0001). After 12 weeks, 85% of patients had achieved remission. Six-week and 12-week BOT administration were safe and well tolerated; 5% of patients who received BOT developed symptomatic, mild candida, which was easily treated with an oral antifungal agent.

**CONCLUSIONS:** In a randomized trial of adults with active EoE, we found that orodispersible effervescent tablets comprising budesonide were significantly more effective than placebo in inducing clinical and histologic remission.

### Example 2

### EOS-2 maintenance of remission trial

**BACKGROUND & AIMS:** Eosinophilic esophagitis (EoE) is a chronic inflammatory disorder. Swallowed topical-acting corticosteroids are effective in bringing active EoE into remission. However, it is not clear whether these drugs are effective for long-term maintenance of remission.

**METHODS:** EOS-2 was a double-blind trial to compare the efficacy and safety of 2 dosages of an orodispersible effervescent tablet comprising budesonide (BOT; now called ,budesonide orodispersible tablet') vs placebo in maintaining remission of EoE over 48 weeks, followed by a 2-years open-label extension phase for continous maintenance therapy with recommended BOT 0.5mg BID (if needed dose could be increased to BOT 1mg BID). Maintenance of remission was defined as absence of clinical and histologic relapse and no premature withdrawal for any reason. Two hundred and four adults with EoE in clinical and histologic remission, from 29 European study sites, were randomly assigned to groups given BOT 0.5 mg twice daily (n= 68), BOT 1.0 mg twice daily (n=68), or placebo twice daily (n=68) for up to 48 weeks.

**RESULTS:** At end of double-blind 48-weeks treatment, 73.5% of patients receiving BOT 0.5 mg twice daily and 75% receiving BOT 1.0 mg twice daily were in persistent remission compared with 4.4% of patients in the placebo group (P < .001 for both comparisons of BOT with placebo). Median time to relapse in the placebo group was 87 days. The frequency of adverse events was similar in the BOT and placebo groups. Morning serum levels of cortisol were in the normal range at baseline and did not significantly change during treatment. Four patients receiving BOT developed asymptomatic, low serum levels of cortisol. Clinically manifested candidiasis was suspected in 16.2% of patients in the BOT 0.5 mg group and in 11.8% of patients in the BOT 1.0 mg group; all infections resolved with treatment.

**CONCLUSIONS:** In a phase 3 trial, up to 48 weeks of treatment with BOT (0.5 mg or 1.0 mg twice daily) was superior to placebo in maintaining remission of EoE. Both dosages were equally effective and well tolerated.

## Claims

1. Orodispersible effervescent tablet comprising budesonide for use in the treatment of eosinophilic esophagitis **characterized in that** a complete mucosal healing is achieved by the administration twice daily of the orodispersible effervescent tablet for at least six weeks of treatment and could be maintained up 144 weeks.

2. Orodispersible effervescent tablet for use according to claim 1 **characterized in that** the orodispersible tablet comprises 0.5 mg budesonide.

3. Orodispersible effervescent tablet for use according to claim 1 **characterized in that** the orodispersible tablet comprises 1 mg budesonide.

4. Orodispersible effervescent tablet for use according to claim 1 **characterized in that** the orodispersible tablet comprises 2 mg budesonide and is administered once daily.

5. Orodispersible effervescent tablet for use according to any of claims 1 to 4 **characterized in that** the deep endoscopic remission is defined as 0 points of the endoscopic reference score.

6. Orodispersible effervescent tablet for use according to claims 1, 2 and 5 **characterized in that** an orodispersible tablet comprising 0.5 mg budesonide is administered twice daily for a period of 6-12 weeks in order to achieve induction of the complete mucosal healing.

7. Orodispersible effervescent tablet for use according to claims 1, 3 and 5 **characterized in that** an orodispersible tablet comprising 1 mg budesonide is administered twice daily for a period of 6-12 weeks in order to achieve induction of the complete mucosal healing.

8. Orodispersible effervescent tablet for use according to any of claims 1 to 7 **characterized in that** the complete mucosal healing is reflected in increased esophageal distensibility.

9. Orodispersible effervescent tablet for use according to any of claims 1 to 8 **characterized in that** the dissolution of the orodispersible effervescent tablet in the mouth occurs in a time span of 2 minutes up to 20 minutes by the action of saliva.

10. Orodispersible effervescent tablet for use according to any of claims 1 to 9 **characterized in that** the dissolution of the orodispersible effervescent tablet in the mouth occurs in a time span of 2 minutes up to 5 minutes by the action of saliva.

11. Orodispersible effervescent tablet for use according to claim 1 and 3 **characterized in that** the daily dosage of 2 mg is administered as one tablet comprising 1.0 mg budesonide in the morning and a further tablet comprising 1.0 mg budesonide in the evening.

12. Orodispersible effervescent tablet for use according to any of claims 1 to 11 wherein the complete mucosal healing exhibits an acceptable esophageal distensibility.

13. Orodispersible effervescent tablet for use according to any of claims 1 and 4 **characterized in that** the daily dosage is 2 mg budesonide, which is administered in the maintenance phase.

14. Orodispersible effervescent tablet for use according to any of claims 1 to 13 wherein the maintenance treatment is performed for a time span ranging from six weeks up to three years.
